# EUROPEAN PATENT APPLICATION

(11) **EP 4 163 368 A1**
(43) Date of publication of application: **12.04.2023**
(21) Application number: 20939965.8
(22) Date of filing: 08.07.2020
(51) Int. Cl.: C12N 9/02, C12N 15/53, C12N 15/70, C12N 1/21, C12P 7/02, C12R 1/19

(54) **KETOREDUCTASE MUTANT AND APPLICATION THEREOF**

(30) Priority: 08.06.2020 CN 202010510049
(71) Applicant: Asymchem Life Science (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); LIU, Fang, Tianjin 300457 (CN); WANG, Zujian, Tianjin 300457 (CN); SHI, Wanming, Tianjin 300457 (CN); YAN, Junjie, Tianjin 300457 (CN); ZHANG, Mujiao, Tianjin 300457 (CN)
(74) Representative: Zacco GmbH
(86) International application number: PCT/CN2020/100913
(87) International publication number: WO 2021/248602

(57) **Abstract**

Provided is a ketoreductase mutant and applications thereof. Compared with an amino acid sequence shown in SEQ ID NO: 1, the amino acid sequence of the ketoreductase mutant includes at least one of the following mutation sites: L198P, S96W/I/L/V, M194L or L1526, herein "/" means "or." The ketoreductase mutant provides high selectivity and high activity with respect to a substrate ketone, and the application of the mutant in a catalytic reduction reaction of a ketone substrate may increase the production efficiency of a chiral alcohol compound corresponding thereto.

## Description

### Technical Field

The present invention relates to the field of enzyme production, in particular to a ketoreductase mutant and an application thereof.

### Background

Chiral alcohol, as an important intermediate, is widely used in synthesis of chiral drugs and other chiral fine chemicals, and there are many related synthetic reports already at home and abroad. Many chiral drugs contain one or more chiral centers, and the pharmacological activities, metabolic processes, metabolic rates, and toxicities of the different chiral drugs are significantly different. Usually one enantiomer is effective, and the other enantiomer is low-effective or ineffective, or even toxic. Therefore, how to efficiently and stereoselectively construct compounds containing the chiral centers is of great significance in pharmaceutical research and development.

There are many types of production of the chiral alcohol. For example, racemic alcohol may be used as a starting raw material to obtain alcohol with a single configuration by kinetic resolution. However, the theoretical yield of this method is 50%, and the cost is relatively high. The chiral alcohol may be synthesized by asymmetric catalytic hydrogenation using chiral oxazolborane hydroboration, chiral transition metal rhodium, rubidium and the like. However, the reaction conditions are relatively severe, such as the high temperature and the high pressure, and the residual problem of toxic reagents such as transition metals is involved.

A ketoreductase is used as a catalyst to replace traditional chemical catalysts, it has the advantages of mild reaction and less environmental pollution and the like, and receives a great attention in the pharmaceutical field.

Biocatalysis accords with the concept of sustainable development and green chemistry. Biocatalysts are derived from renewable materials, and play a catalytic role under the mild conditions. The different biocatalysts catalyze reactions under the similar condition, and are easy to design as a more economical cascade catalytic reaction. Therefore, the use of the ketoreductase to catalyze the synthesis of the chiral alcohol is an extremely potential catalytic mode, but there are also certain difficulties, especially for different substrates, there are problems such as the low catalytic activity of the enzyme, and low product selectivity after enzyme catalysis. Therefore, it is of great significance to develop the ketoreductase with high chiral specificity and high activity.

### Summary

A main purpose of the present invention is to provide a ketoreductase mutant and an application thereof, as to solve a problem in the prior art that the selectivity of a substrate catalyzed by a ketoreductase or a mutant thereof is relatively low.

In order to achieve the above purpose, according to one aspect of the present invention, a ketoreductase mutant is provided, and compared with an amino acid sequence shown in SEQ ID NO: 1, the amino acid sequence of the ketoreductase mutant includes at least one of the following mutation sites: L198P, S96W/I/L/V, M194L or L152G, herein "/" represents "or".

Further, the amino acid sequence of the ketoreductase mutant further includes one of the following mutation sites: Q24L, V34M, I35V, A72V, L80R, T94S, Y95F, V99T, S101N, L104S, I143V, I147V, A153T, Y178C, V185I, Y189F/W, I190T, P193H, V195I, T199I, E201G/D/H, D202A, A203G/S, R206Q, D234V, K237E and T240I; or the ketoreductase mutant has a mutation site in an mutated amino acid sequence, and has more than 80% of the identity with the mutated amino acid sequence, preferably more than 85%, more preferably more than 90%, and further preferably more than 95% of the identity with the mutated amino acid sequence.

Further, the amino acid sequence of the ketoreductase mutant has a mutation site of any one of the following combinations:
S96W+L198P, , S96L+L198P, S96W+M194L+L198P, S96W+Y189F+L198P, S96W+L198P+E201D, S96W+I143V+M194L+L198P+R206Q, S96W+I143V+M194L+L198P+E201D+R206Q, S96W+I143V+I190T+M194L+L198P+E201D+R206Q, T94S+S96W+M194L+L198P, S96W+M194L+L198P+E201G, T94S+S96I+M194L+L198P, T94S+S96W+I143V+I190T+M194L+L198P+E201D+R206Q, S96W+I143V+I190T+M194L+L198P+E201G+R206Q, T94S+S96W+I143V+I190T+M194L+L198P+E201G+R206Q, T94S+Y95F+S96W+I143V+I190T+M194L+L198P+E201D+R206Q, T94S+Y95F+S96W+I143V+I190T+M194L+L198P+E201G+R206Q, S96W+I143V+I190T+M194L+V195I+L198P+E201D+R206Q, S96W+I147V+M194L+L198P, S96W+A153T+M194L+L198P, Q24L+A72V+S101N+S96W+M194L+L198P, S96W+I190T+M194L+L198P+D234V, S96W+M194L+L198P+K237E, S96W+P193H+M194L+L198P, V34M+S96W+M194L+L198P, S96W+M194L+L198P+E201H, L80R+S96W+M194L+L198P, S96W+M194L+L198P+D234V, S96W+I143V+I190T+M194L+L198P+T199I+E201D+R206Q, S96W+V99T+I143V+I190T+M194L+L198P+E201D+R206Q, S96W+L104S+I143V+I190T+M194L+L198P+E201D+R206Q, T94S+Y95F+S96W+I143V+I190T+M194L+V1951+L198P+E201G+R206Q, T94S+Y95F+S96W+I143V+I190T+M194L+V195I+L198P+E201D+R206Q, I35V+T94S+Y95F+S96W+I143V+I190T+M194L+L198P+E201G+R206Q, T94S+Y95F+S96W+I143V+V185I+I190T+M194L+L198P+E201G+R206Q, T94S+Y95F+S96W+I143V+I190T+M194L+L198P+E201G+R206Q+T240I, T94S+Y95F+S96W+I143V+Y178C+I190T+M194L+V195I+L198P+E201D+R206Q, T94S+Y95F+S96W+I143V+I190T+M194L+V195I+L198P+E201D+A203G+R206Q, S96W+M194L+I143V+R206Q+I190T+L198P+E201D+T94S+Y95F+V195I+A203S, T94S+Y95F+S96W+I143V+Y189W+I190T+M194L+L198P+E201G+R206Q, T94S+Y95F+S96W+M194L+L198P+I143V+R206Q+I190T+E201D+D201G+V195I+Y189W, T94S+Y95F+S96W+I143V+Y189W+I190T+M194L+V195I+L198P+E201D+R206Q, T94S+Y95F+S96W+I143V+Y178C+Y189W+I190T+M194L+V195I+L198P+E201D+R206Q, T94S+Y95F+S96W+I143V+Y189W+I190T+M194L+V195I+L198P+E201D+A203G+R206Q, T94S+Y95F+S96W+I143V+R206Q+Y189W+I190T+M194L+V195I+L198P+E201D+A203S, T94S+Y95F+S96W+I143V+Y178C+I190T+M194L+V195I+L198P+E201D+D202A+A203G+R206Q ,or T94S+Y95F+S96W+I143V+Y178C+Y189W+1190T+M194L+V195I+L198P+E201D+D202A+A203 G+R206Q.

In order to achieve the above purpose, according to a second aspect of the present invention, a DNA molecule is provided, and the DNA molecule encodes any one of the above ketoreductase mutants.

According to a third aspect of the present invention, a recombinant vector is provided, and the recombinant vector is linked to the DNA molecule.

According to a fourth aspect of the present invention, a host cell is provided, and the host cell contains any one of the above recombinant vectors.

Further, the host cell includes a prokaryotic cell or a eukaryotic cell; and preferably the prokaryotic cell is *Escherichia coli.*

According to a fifth aspect of the present invention, a method for generating chiral alcohol is provided, and the method includes: a step of using any of the above ketoreductase mutants to catalyze a reduction reaction of a chiral ketone compound represented in Formula (I) to generate the chiral alcohol.

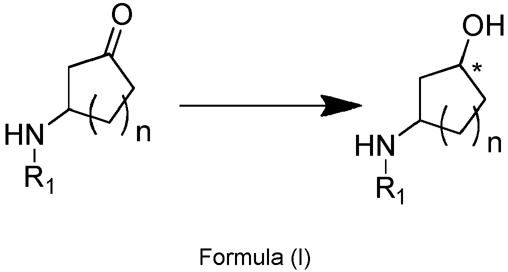

Herein, n is 0, 1, 2 or 3; R1 is selected from: H, OH, a substituted or unsubstituted C₁₋₁₀ alkyl, a substituted or unsubstituted C₃₋₆ cycloalkyl, a substituted or unsubstituted C₆₋₁₂ aryl, a substituted or unsubstituted C₃₋₆ heterocyclyl; or, R1 is a benzene ring together with a linked heterocyclyl to form a fused ring system.

Further, the chiral ketone compound represented in Formula (I) is

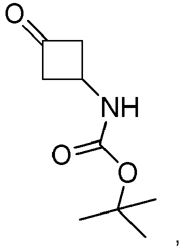

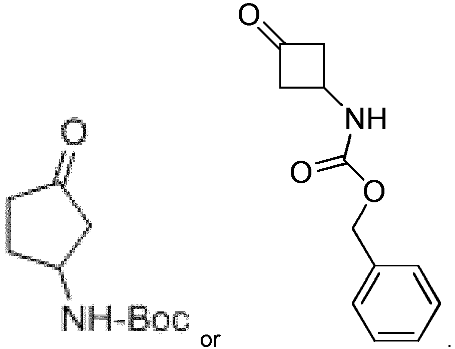

Further, the mass ratio of the ketoreductase mutant to the chiral ketone compound is 0.1-10:1; preferably, the concentration of the ketone compound in the reduction reaction system is 1 g/L~100 g/L; and preferably, the reduction reaction is performed at 20~45°C.

A technical scheme of the present invention is applied, the present application rationally transforms a protein of the existing ketoreductase mutant by a method combined with rational design and random mutation, and uses the substrate ketone of the present application to perform the catalytic activity and stereoselective screening on the obtained mutant, finally mutant strains with high selectivity and high activity are obtained. The strains containing these mutants are applied in the catalytic reduction reaction of the ketone substrate of the present application, which may improve the production efficiency of its corresponding chiral alcohol compounds.

### Detailed Description of the Embodiments

It should be noted that embodiments in the present application and features of the embodiments may be combined with each other in the case without conflicting. The present invention is described in detail below with reference to the embodiments.

"Ketoreductase" and "KRED" are used interchangeably herein, to refer to a polypeptide capable of reducing a ketone group into its corresponding alcohol. Specifically, the ketoreductase of the present application are capable of stereoselectively reducing a ketone compound into a corresponding alcohol product. Such ketoreductases typically use a cofactor reduced nicotinamide adenine dinucleotide (NADH) or reduced nicotinamide adenine dinucleotide nicotine as a reducing agent. In the present application, the ketoreductase refers to naturally existing (wild-type) ketoreductases as well as non-naturally existing ketoreductase mutants generated by artificial treatment.

"Naturally existing" or "wild-type" as opposed to "mutant" refers to a form found in nature. For example, a naturally-existing or wild-type polypeptide or polynucleotide sequence is a sequence that exists in an organism, it may be separated from a source in nature, and is not deliberately modified or changed artificially.

In the present application, reference to, for example, cell, nucleic acid or polypeptide "recombination" means that it is already modified in a manner which does not exist in nature, or in the same form existing in nature, but it is obtained from synthetic materials and/or prepared or derived by the treatment of a recombinant technology, or corresponds to a cell, a nucleic acid or a polypeptide in its native or inherent form. Herein, non-restrictive examples include a recombinant cell expressing a gene in a cell other than in its inherent (non-recombinant) form or expressing an inherent gene at different levels.

"Percentage of sequence identity" refers to a comparison between polynucleotide sequences or amino acid sequences, and is determined by comparing two optimally aligned sequences across a comparison window, herein the portion of the polynucleotide sequences or amino acid sequences within the comparison window may include addition or deletion (namely, a vacancy) compared to the reference sequence, which is used for the optimal alignment of the two sequences. The percentage may be calculated as follows: by determining the number of positions in the two sequences in which the same nucleic acid base or amino acid residue occurs, to produce the number of matched positions, dividing the number of the matched positions by the total number of the positions in the comparison window, and multiplying a result by 100 to obtain the percentage of the sequence identity. Optionally, the percentage may be calculated as follows: by determining the number of the positions in the two sequences in which the same nucleic acid base or amino acid residue occurs or in which a nucleic acid base or amino acid residue is aligned with a vacancy, to produce the number of matched positions, dividing the number of the matched positions by the total number of the positions in the comparison window, and multiplying a result by 100 to obtain the percentage of the sequence identity. Herein, the "reference sequence" refers to a designated sequence used as a basis for sequence comparison. The reference sequence may be a subset of a larger sequence, for example, a segment of a full-length gene or polypeptide sequence.

Site-directed mutagenesis: refers to introduction of desired changes (usually the changes that characterize favorable directions) into a target DNA fragment (either a genome or a vector) by a method such as a polymerase chain reaction (PCR), including addition of bases, deletion, point mutation and the like. The site-directed mutagenesis may rapidly and efficiently improve the characters and representation of a target protein expressed by DNA, and is a very useful means in genetic research work.

In order to obtain more ketone substrates for conversion to its corresponding chiral alcohol products, more ketoreductases need to be developed. The rational transformation of enzymes is to modify a substrate binding site, a coenzyme binding site, a surface and other sites of the enzyme based on the three-dimensional molecular structure of the enzyme, as to change the catalytic properties of the enzyme, and improve the activity and selectivity and other properties of the enzyme.

The directed evolution of the enzyme is an irrational design of the protein, the special evolutionary conditions are artificially created, the natural evolutionary mechanisms are simulated, genes are modified in vitro, technologies such as error-prone PCR and DNA shuffling are applied, and a high-efficiency screening system is combined to obtain a new enzyme with expected properties.

The present application aims to develop a ketoreductase that catalyzes the conversion of the ketone compound substrate of the following structural formula (I) to its corresponding chiral alcohol by combining the rational transformation and directed evolution of the enzyme:

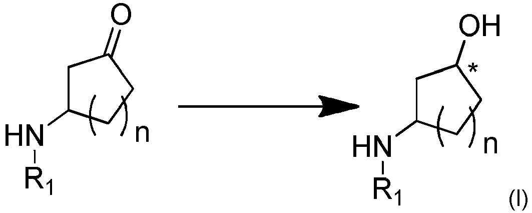

In the above structural formula, n is 0, 1, 2 or 3; R1 is selected from: H, OH, a substituted or unsubstituted C₁₋₁₀ alkyl, a substituted or unsubstituted C₃₋₆ cycloalkyl, a substituted or unsubstituted C₆₋₁₂ aryl, a substituted or unsubstituted C₃₋₆ heterocyclyl; or, R1 is a benzene ring together with a linked heterocyclic ring to form a fused ring system.

The inventor uses the ketone compound represented by the following formula (II) as a substrate, and uses wild-type ketoreductases from more than 170 different species and mutants of some ketoreductases to catalyze the reaction of such substrates, as to detect the catalytic activity and selectivity of the ketoreductases from the different sources. After being detected, the wild-type ketoreductase and some of its mutants have the lower selectivity to the above substrate ketone, a large number of isomers are generated, and the activity is low generally. It is specifically shown in Table 1.

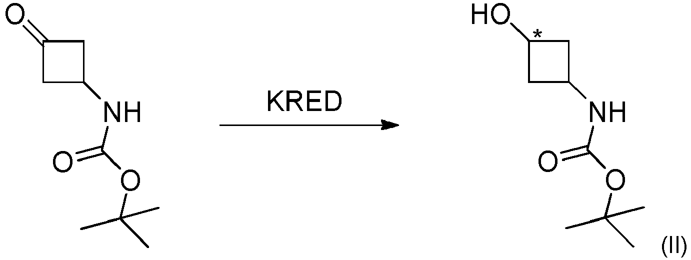

**Table 1:**

| Species source | Activity | Selectivity |
|---|---|---|
| Thermoanaerobacter ethanolicus | ** | - |
| L. Kefir | ** | * |
| Acinetobacter baylyi | - | *** |
| Nocardia globerula | - | *** |
| Acetobacter sp. | * | * |
| Variant 1 is derived from Acetobacter sp. | ** | ** |
| Variant 2 is derived from Acetobacter sp. | - | ** |
| Variant 3 is derived from Acetobacter sp. | - | * |
| Variant 4 is derived from Acetobacter sp. | - | * |
| Variant 5 is derived from Acetobacter sp. | - | ** |
| Variant 6 is derived from Acetobacter sp. | - | * |
| Variant 7 is derived from Acetobacter sp. | - | - |
| Variant 8 is derived from Acetobacter sp. | - | * |
| Variant 9 is derived from Acetobacter sp. | - | * |
| Variant 10 is derived from Acetobacter sp. | - | * |
| Variant 11 is derived from Acetobacter sp. | - | * |
| Variant 12 is derived from Acetobacter sp. | * | * |
| Variant 13 is derived from Acetobacter sp. | * | ** |
| Variant 14 is derived from Acetobacter sp. | - | * |
| Pyrococcus furiosus | - | * |
| Neurospora crassa | * | * |
| Rhodococcus erythropolis | * | * |
| Devosia riboflavina | - | ND |
| Candida macedoniensis | - | ND |
| Candida parapsilosis | - | ND |
| Candida parapsilosis | - | ND |
| Geobacillus thermodenitrificans | - | ND |
| Pichia stipites | - | ND |
| horse liver | - | ND |
| Bacillus sp. | - | ND |
| Bacillrcs stearothermophilus | - | ND |
| Lactobacillus brevis | - | ND |
| Leuconostoc mesenteroides | - | ND |
| horse liver | - | ND |
| Thermoanaerobium brockii | - | ND |
| Lactobacillus kefir | - | ND |
| Streptomyces coelicolor | - | ND |
| Candida maris strain | - | ND |
| Picrophilus torridus | - | ND |
| Nocardia cyriacigeorgica | - | ND |
| Acinetobacter calcoaceticus | - | ND |
| Kluyveromyces aestuarii | - | ND |
| Candida parapsilosis | - | ND |
| Candida magnolia | - | ND |
| Rhodococcus sp. | - | ND |
| Pyrobaculum aerophilum | - | ND |
| Cupriavidus necator | - | ND |
| Escherichia coli K12 strain | - | ND |
| Thermococcus sibiricus | - | ND |
| Saccharomyces cerevisiae | - | ND |
| Rhodococcus ruber | - | ND |
| Candida utilis. | - | ND |
| Acinetobacter sp. and 113 other ketoreductases from different species or its mutants | - | ND |

| | | |
|---|---|---|
| Activity: ** means that after 5 mass ratios of the enzyme are used, and 50 reaction volumes are catalyzed for 16 hours, the conversion rate is 80%~90%; * means that after 5 mass ratios of the enzyme are used, and 50 reaction volumes are catalyzed for 16 hours, the conversion rate is 30%~80%; - means that after 5 mass ratios of the enzyme are used, and 50 reaction volumes are catalyzed for 16 hours, the conversion rate is 5%~30%; ND means that after 5 mass ratios of the enzyme are used, and 50 reaction volumes are catalyzed for 16 hours, the conversion rate is less than 5% or a product is not detected; Selectivity: - means that the selectivity is less than 5%; * means that the selectivity is 5%~30%; ** means that the selectivity is 30%~60%; *** means that the selectivity is 60%~90%; **** means that the selectivity is 90%~95%; and ***** means that the selectivity is 95%~100%. | | |

In the above table, the ketoreductases from the different species refer to the wild-type enzyme, and its specific gene ID numbers may be found from National Center of Biotechnology Information (NCBI). Each variant is a mutant obtained by mutating a wild-type ketoreductase from the same species at different sites. The differences between the above variants 1 to 14 derived from Acetobacter sp and the mutation sites of the wild-type ketoreductase from Acetobacter sp are specifically shown in the following table:

**Table 2:**

| Mutant strain | Mutation site(s) |
|---|---|
| Variant 1 | A94T (corresponding to SEQ ID NO:1) |
| Variant 2 | E144S+A94T |
| Variant 3 | E144S+N156T |
| Variant 4 | E144S+N156S |
| Variant 5 | E144S+A94T+N156T |
| Variant 6 | E144S |
| Variant 7 | N156T |
| Variant 8 | E144S+A94N-N156V |
| Variant 9 | E144S |
| Variant 10 | E144A+L152F |
| Variant 11 | E144A+L152Y+L198Q+E201G+G6S+L146M+I147V+D42E |
| Variant 12 | E144A+L152C |
| Variant 13 | E144A+L152Y+L198Q+E201G+G6S+L146M+I147V+D42E+ T199V+F165Y+K200H |
| Variant 14 | E144A+L152Y+L198Q+E201G+G6S+L146M+I147V+D42E+ T199V+S96N+N156S |

As described above, the ketoreductase AcCR derived from Acetobacter pasteurianus may catalyze the synthesis of the chiral alcohol, but whether it is the wild-type ketoreductase AcCR or its mutants, the selectivity for the above types of the substrate ketones is low, and the activity is lower, a large amount of the enzyme is required for catalysis, the chiral purity of the obtained product is extremely low, the difficulty of post-treatment is increased, the yield is low, and the steps are complicated.

The present invention aims at the above deficiencies of the ketoreductase AcCR, in order to further obtain a ketoreductase with the higher catalytic activity and/or selectivity, the present application evolves the above ketoreductase AcCR variant 1 derived from Acetobacter pasteurianus, transforms the AcCR protein by the rational design, and acquires the mutant protein by random mutation, and the beneficial mutant is targeting screened by a high-throughput screening method.

The present application is based on the ketoreductase variant 1 derived from Acetobacter pasteurianus (referred to as a starting enzyme or an initiating enzyme in the present application, and its specific amino acid sequence is SEQ ID NO: 1) as a basis for mutation transformation, and adopts a method combined with the rational design and random mutation to rationally transform the AcCR starting enzyme, as to improve its selectivity and activity properties. The substrate ketone represented by the previous structural formula (II) is used to perform catalytic activity verification on the obtained mutant, and finally the mutant strain with the high selectivity and high activity is obtained, it may be applied to the industrial production conditions and generate the chiral alcohol with the high chiral purity.
SEQ ID NO: 1:

MARVAGKVAIVSGAANGIGKATAQLLAKEGAKVVIGDLKEEDGQKAVAEIKAAGGEAAFVKLNV
TDEAAWKAAIGQTLKLYGRLDIAVNNAGITYSGSVESTSLEDWRRVQSINLDGVFLGTQVAIEAMKK
SGGGSIVNLSSIEGLIGDPMLAAYNASKGGVRLFTKSAALHCAKSGYKIRVNSVHPGYIWTPMVAG
LTKEDAAARQKLVDLHPIGHLGEPNDIAYGILYLASDESKFVTGSELVIDGGYTAQ.

The technical scheme of the present invention transforms the AcCR starting enzyme protein by the technology combined with the rational design and random mutation, the catalytic activity of the mutant (see Table 3) is significantly improved, and the selectivity of the mutant (see Table 4) is significantly enhanced, so it is suitable for scale-up production of the high-purity chiral alcohol.

**Table 3:**

| Mutant strain | Mutation site | Activity |
|---|---|---|
| Starting enzyme | Null | ** |
| M1 | S96W | ** |
| M2 | L152G | *** |
| M3 | L198P | *** |
| M4 | S96W+L198P | **** |
| M5 | S96L+L198P | **** |
| M6 | S96W+M194L+L198P | *** |
| M7 | S96W+Y189F+L198P | **** |
| M8 | S96W+L198P+E201D | ****** |
| M9 | S96W+I143V+M194L+L198P+R206Q | **** |
| M10 | S96W+I143V+M194L+L198P+E201D+R206Q | ***** |
| M11 | S96W+I143V+I190T+M194L+L198P+E201D+R206Q | ****** |
| M12 | T94S+S96W+M194L+L198P | **** |
| M13 | S96W+M194L+L198P+E201G | ***** |
| M14 | T94S+S96I+M194L+L198P | **** |
| M15 | T94S+S96W+I143V+I190T+M194L+L198P+E201D+R206Q | ****** |
| M16 | S96W+I143V+I190T+M194L+L198P+E201G+R206Q | ****** |
| M17 | T94S+S96W+I143V+I190T+M194L+L198P+E201G+R206Q | ****** |
| M18 | T94S+Y95F+S96W+I143V+I190T+M194L+L198P+E201D+R206Q | ****** |
| M19 | T94S+Y95F+S96W+I143V+I190T+M194L+L198P+E201G+R206Q | ****** |
| M20 | S96W+I143V+I190T+M194L+V195I+L198P+E201D+R206Q | ***** |
| M21 | S96W+I147V+M194L+L198P | **** |
| M22 | S96W+A153T+M194L+L198P | **** |
| M23 | Q24L+A72V+S101N+S96W+M194L+L198P | **** |
| M24 | S96W+I190T+M194L+L198P+D234V | **** |
| M25 | S96W+M194L+L198P+K237E | **** |
| M26 | S96W+P193H+M194L+L198P | **** |
| M27 | V34M+S96W+M194L+L198P | **** |
| M28 | S96W+M194L+L198P+E201H | ***** |
| M29 | L80R+S96W+M194L+L198P | **** |
| M30 | S96W+M194L+L198P+D234V | **** |
| M31 | S96W+I143V+I190T+M194L+L198P+T199I+E201D+R206Q | **** |
| M32 | S96W+V99T+I143V+I190T+M194L+L198P+E201D+R206Q | **** |
| M33 | S96W+L104S+I143V+I190T+M194L+L198P+E201D+R206Q | ***** |
| M34 | T94S+Y95F+S96W+I143V+I190T+M194L+V1951+L198P+E201G+R2 06Q | ****** |
| M35 | T94S+Y95F+S96W+I143V+I190T+M194L+V195I+L198P+E201D+R2 06Q | ****** |
| M36 | I35V+T94S+Y95F+S96W+I143V+I190T+M194L+L198P+E201G+R20 6Q | **** |
| M37 | T94S+Y95F+S96W+I143V+V185I+I190T+M194L+L198P+E201G+R2 06Q | ***** |
| M38 | T94S+Y95F+S96W+I143V+I190T+M194L+L198P+E201G+R206Q+T 240I | ***** |
| M39 | T94S+Y95F+S96W+I143V+Y178C+I190T+M194L+V195I+L198P+E2 01D+R206Q | ***** |
| M40 | T94S+Y95F+S96W+I143V+I190T+M194L+V195I+L198P+E201D+A2 03G+R206Q | ***** |
| M41 | L198P+S96W+M194L+I143V+R206Q+I190T+E201D+T94S+Y95F+V 195I+A203S | ***** |
| M42 | T94S+Y95F+S96W+I143V+Y189W+I190T+M194L+L198P+E201G+R 206Q | ***** |
| M43 | T94S+Y95F+S96W+M194L+L198P+1143 V+ R206Q+1190T+E201D+D 201G+V195I+Y189W | ***** |
| M44 | T94S+Y95F+S96W+I143V+YI89W+I190T+M194L+V195I+L198P+E20 1D+R206Q | ***** |
| M45 | T94S+Y95F+S96W+I143V+Y178C+Y189W+I190T+M194L+V1951+L1 98P+E201D+R206Q | ***** |
| M46 | T94S+Y95F+S96W+I143V+Y189W+I190T+M194L+V195I+L198P+E2 01D+A203G+R206Q | ***** |
| M47 | T94S+Y95F+S96W+I143V+R206Q+Y189W+I190T+M194L+V195I+L1 98P+E201D+A203S | ***** |
| M48 | T94S+Y95F+S96W+I143V+Y178C+I190T+M194L+V195I+L198P+E2 01D+D202A+A203G+R206Q | ***** |
| M49 | L198P+T94S+Y95F+S96W+I143V+Y178C+Y189W+I190T+M194L+V 195I+E201D+D202A+A203G+R206Q | ***** |

In the above table, * means that after 0.5 mass ratios of the enzyme ( ) are used, and 50 reaction volumes are catalyzed for 16 hours, the conversion rate is less than 20%; ** means that after 0.5 mass ratios of the enzyme are used, and 50 reaction volumes are catalyzed for 16 hours, the conversion rate is 30% %~50%; *** means that after 0.5 mass ratios of the enzyme are used, and 50 reaction volumes are catalyzed for 16 hours, the conversion rate is greater than 50%; **** means that after 0.2 mass ratios of the enzyme are used, and 20 reaction volumes are catalyzed for 16 hours, the conversion rate is 0%~50%; ***** means that after 0.2 mass ratios of the enzyme are used, and 50 reaction volumes are catalyzed for 16 hours, the conversion rate is 50%~90%; ****** means that after 0.2 mass ratios of the enzyme are used, and 50 reaction volumes are catalyzed for 16 hours, the conversion rate is 90%~100%.

**Table 4:**

| Mutant strain | Mutation site | Selecti vity |
|---|---|---|
| Starting enzyme | Null | * |
| M1 | S96W | *** |
| M2 | L152G | - |
| M3 | L198P | *** |
| M4 | S96W+L198P | *** |
| M5 | S96L+L198P | *** |
| M6 | S96W+M194L+L198P | **** |
| M7 | S96W+Y189F+L198P | *** |
| M8 | S96W+L198P+E201D | *** |
| M9 | S96W+I143V+M194L+L198P+R206Q | **** |
| M10 | S96W+I143V+M194L+L198P+E201D+R206Q | **** |
| M11 | S96W+I143V+I190T+M194L+L198P+E201D+R206D | **** |
| M12 | T94S+S96W+M194L+L198P | **** |
| M13 | S96W+M194L+L198P+E201G | **** |
| M14 | T94S+S96I+M194L+L198P | **** |
| M15 | T94S+S96W+I143V+I190T+M194L+L198P+E201D+R206Q | ***** |
| M16 | S96W+I143V+I190T+M194L+L198P+E201G+R206Q | **** |
| M17 | T94S+S96W+I143V+I190T+M194L+L198P+E201G+R206Q | ***** |
| M18 | T94S+Y95F+S96W+I143V+I190T+M194L+L198P+E201D+R206Q | ***** |
| M19 | T94S+Y95F+S96W+I143V+I190T+M194L+L198P+E201G+R206Q | ***** |
| M20 | S96W+I143V+I190T+M194L+V195I+L198P+E201D+R206Q | ***** |
| M21 | S96W+I147V+M194L+L198P | **** |
| M22 | S96W+A153T+M194L+L198P | **** |
| M23 | Q24L+A72V+S101N+S96W+M194L+L198P | **** |
| M24 | S96W+I190T+M194L+L198P+D234V | **** |
| M25 | S96W+M194L+L198P+K237E | **** |
| M26 | S96W+P193H+M194L+L198P | **** |
| M27 | V34M+S96W+M194L+L198P | **** |
| M28 | S96W+M194L+L198P+E201H | **** |
| M29 | L80R+S96W+M194L+L198P | **** |
| M30 | S96W+M194L+L198P+D234V | ***** |
| M31 | S96W+I143V+I190T+M194L+L198P+T199I+E201D+R206Q | ***** |
| M32 | S96W+V99T+I143V+I190T+M194L+L198P+E201D+R206Q | **** |
| M33 | S96W+L104S+I143V+I190T+M194L+L198P+E201D+R206Q | **** |
| M34 | T94S+Y95F+S96W+I143V+I190T+M194L+V195I+L198P+E201G+R2 06Q | ***** |
| M35 | T94S+Y95F+S96W+I143V+I190T+M194L+V195I+L198P+E201D+R2 | ***** |
| | 06Q | |
| M36 | I35V+T94S+Y95F+S96W+I143V+I190T+M194L+L198P+E201G+R20 6Q | ***** |
| M37 | T94S+Y95F+S96W+I143V+V185I+I190T+M194L+L198P+E201G+R2 06Q | ***** |
| M38 | T94S+Y95F+S96W+I143V+I190T+M194L+L198P+E201G+R206Q+T 240I | ***** |
| M39 | T94S+Y95F+S96W+I143V+Y178C+I190T+M194L+V195I+L198P+E2 01D+R206Q | ***** |
| M40 | T94S+Y95F+S96W+I143V+I190T+M194L+V195I+L198P+E201D+A2 03G+R206Q | ***** |
| M41 | L198P+S96W+M194L+I143V+R206Q+I190T+E201D+T94S+Y95F+V 195I+A203S | ***** |
| M42 | T94S+Y95F+S96W+I143V+Y189W+I190T+M194L+L198P+E201G+R 206Q | ***** |
| M43 | T94S+Y95F+S96W+M194L+L198P+1143 V+ R206Q+1190T+ E201D+D 201G+V195I+Y189W | ***** |
| M44 | T94S+Y95F+S96W+I143V+YI89W+I190T+M194L+V195I+L198P+E20 1D+R206Q | ***** |
| M45 | T94S+Y95F+S96W+I143V+Y178C+Y189W+I190T+M194L+V195I+L1 98P+E201D+R206Q | ***** |
| M46 | T94S+Y95F+S96W+I143V+Y189W+I190T+M194L+V195I+L198P+E2 01D+A203G+R206Q | ***** |
| M47 | T94S+Y95F+S96W+I143V+R206Q+Y189W+I190T+M194L+V195I+L1 98P+E201D+A203S | ***** |
| M48 | T94S+Y95F+S96W+I143V+Y178C+I190T+M194L+V195I+L198P+E2 01D+D202A+A203G+R206Q | ***** |
| M49 | L198P+T94S+Y95F+S96W+I143V+Y178C+Y189W+I190T+M194L+V | ***** |
| | 195I+E201D+D202A+A203G+R206Q | |

| | | |
|---|---|---|
| In the above table, - means that the selectivity is less than 5%; * means that the selectivity is 5%~40%; *** means that the selectivity is 40%~90%; **** means that the selectivity is 90%~95%; and ***** means that the selectivity is 95%~100%. | | |

Therefore, on the basis of the above research results, the applicant proposes a technical scheme of the present application. In a typical embodiment of the present application, a ketoreductase mutant is provided, and compared with an amino acid sequence shown in SEQ ID NO: 1, the amino acid sequence of the ketoreductase mutant includes at least one of the following mutation sites: L198P, S96W/I/L/V, M194L, L152G, herein "/" represents "or". The mutant of the present invention may use the ketone compound represented by the structural formula (I) as a substrate, and may efficiently generate a corresponding chiral alcohol compound by stereoselective reduction, and the catalytic activity is greatly improved, which is suitable for promotion of the industrial production of such chiral alcohol.

In a preferred embodiment, the amino acid sequence of the ketoreductase mutant further includes one of the following mutation sites: Q24L, V34M, I35V, A72V, L80R, T94S, Y95F, V99T, S101N, L104S, I143V, I147V, A153T, Y178C, V185I, Y189F/W, I190T, P193H, V195I, T199I, E201G/D/H, D202A, A203G/S, R206Q, D234V, 237E and T240I; or the ketoreductase mutant has a mutation site in an mutated amino acid sequence, and has more than 80% of the identity with the mutated amino acid sequence, preferably more than 85%, more preferably more than 90%, and further preferably more than 95% of the identity with the mutated amino acid sequence. The above ketoreductase mutant may further improve the catalytic activity of the enzyme and the stereoselectivity to the substrate.

In a more preferred embodiment, the amino acid sequence of the ketoreductase mutant has a mutation site of any one of the following combinations: S96W+L198P, , S96L+L198P, S96W+M194L+L198P, S96W+Y189F+L198P, S96W+L198P+E201D, S96W+I143V+M194L+L198P+R206Q, S96W+I143V+M194L+L198P+E201D+R206Q, S96W+I143V+I190T+M194L+L198P+E201D+R206Q, T94S+S96W+M 194L+L198P, S96W+M194L+L198P+E201G, T94S+S96I+M194L+L198P, T94S+S96W+I143V+I190T+M194L+L198P+E201D+R206Q, S96W+I143V+I190T+M194L+L198P+E201G+R206Q, T94S+S96W+I143V+I190T+M194L+L198P+E201G+R206Q, T94S+Y95F+S96W+I143V+I190T+M194L+L198P+E201D+R206Q, T94S+Y95F+S96W+I143V+I190T+M194L+L198P+E201G+R206Q, S96W+I143V+I190T+M194L+V195I+L198P+E201D+R206Q, S96W+I147V+M194L+L198P, S96W+A153T+M194L+L198P, Q24L+A72V+S101N+S96W+M194L+L198P, S96W+I190T+M194L+L198P+D234V, S96W+M194L+L198P+K237E, S96W+P193H+M194L+L198P, V34M+S96W+M194L+L198P, S96W+M194L+L198P+E201H, L80R+S96W+M194L+L198P, S96W+M194L+L198P+D234V, S96W+I143V+I190T+M194L+L198P+T199I+E201D+R206Q, S96W+V99T+I143V+I190T+M194L+L198P+E201D+R206Q, S96W+L104S+I143V+I190T+M194L+L198P+E201D+R206Q, T94S+Y95F+S96W+I143V+I190T+M194L+V195I+L198P+E201G+R206Q, T94S+Y95F+S96W+I143V+I190T+M194L+V195I+L198P+E201D+R206Q, I35V+T94S+Y95F+S96W+I143V+I190T+M194L+L198P+E201G+R206Q, T94S+Y95F+S96W+I143V+V185I+1190T+M194L+L198P+E201G+R206Q, T94S+Y95F+S96W+I143V+I190T+M194L+L198P+E201G+R206Q+T240I, T94S+Y95F+S96W+I143V+Y178C+I190T+M194L+V195I+L198P+E201D+R206Q, T94S+Y95F+S96W+I143V+I190T+M194L+V195I+L198P+E201D+A203G+R206Q, S96W+M194L+I143V+R206Q+I190T+E201D+T94S+Y95F+V195I+L198P+A203S, T94S+Y95F+S96W+I143V+Y189W+I190T+M194L+L198P+E201G+R206Q, T94S+Y95F+S96W+M194L+L198P+I143V+R206Q+I190T+E201D+D201G+V195I+Y189W, T94S+Y95F+S96W+I143V+Y189W+I190T+M194L+V195I+L198P+E201D+R206Q, T94S+Y95F+S96W+I143V+Y178C+Y189W+I190T+M194L+V195I+L198P+E201D+R206Q, T94S+Y95F+S96W+I143V+Y189W+I190T+M194L+V195I+L198P+E201D+A203G+R206Q, T94S+Y95F+S96W+I143V+R206Q+Y189W+I190T+M194L+V195I+L198P+E201D+A203S, T94S+Y95F+S96W+I143V+Y178C+I190T+M194L+V195I+L198P+E201D+D202A+A203G+R206Q or T94S+Y95F+S96W+I143V+Y178C+Y189W+I190T+M194L+V195I+ L198P+E201D+D202A+A203G+R206Q.

In a second exemplary embodiment of the present application, a DNA molecule is provided, and the DNA molecule encodes any one of the above ketoreductase mutants. The above ketoreductase encoded by the DNA molecule has the good catalytic activity and stereoselectivity for the ketone substrate represented by the structural formula (I).

The above DNA molecule of the present invention may also exist in the form of an "expression cassette". The "expression cassette" refers to a linear or circular nucleic acid molecule encompassing DNA and RNA sequences capable of directing the expression of a specific nucleotide sequence in an appropriate host cell. In general, a promoter operably linked to a target nucleotide is included, optionally it is operably linked to a termination signal and/or other regulatory elements. The expression cassette may also include a sequence required for proper translation of the nucleotide sequence. A coding region usually encodes a target protein, but also encodes a target functional RNA in either sense or antisense direction, for example, antisense RNA or untranslated RNA. The expression cassette containing a target polynucleotide sequence may be chimeric, it means that at least one of its components is heterologous to at least one of its other components. The expression cassettes may also be naturally existent, but obtained with efficient recombinant formation for heterologous expression.

In a third exemplary embodiment of the present application, a recombinant vector is provided, and the recombinant vector contains any one of the above DNA molecules. The DNA molecule in the above recombinant vector is placed in an appropriate position of the recombinant vector, so that the above DNA molecule may be correctly and smoothly replicated, transcribed or expressed.

Although a term "containing" is used in the present invention to define the above DNA molecule, it does not mean that other sequences irrelevant to its functions may be arbitrarily added to both ends of the DNA sequence. Those skilled in the art know that, in order to meet the requirements of a recombination operation, it is necessary to add suitable enzyme cleavage sites of a restriction endonuclease at both ends of the DNA sequence, or additionally add a promoter codon, a stop codon and the like. Therefore, if the closed-form expression is used to define, these situations may not be truly covered.

A term "vector" as used in the present invention includes any vectors, cosmids, bacteriophages or agrobacterium binary nucleic acid molecules in double-stranded or single-stranded linear or circular form, preferably a recombinant expression vector, it may be a prokaryotic expression vector or a eukaryotic expression vector, but preferably the prokaryotic expression vector. In some implementation schemes, the recombinant vector is selected from pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18 or pUC-19. More preferably, the above recombinant vector is pET-22b(+).

In a fourth exemplary embodiment of the present application, a host cell is provided, and the host cell contains any one of the above recombinant vectors. The host cell suitable for use in the present invention includes, but is not limited to, a prokaryotic cell, yeast or a eukaryotic cell. Preferably the prokaryotic cell is eubacteria, for example Gram-negative or Gram-positive bacteria. More preferably, the prokaryotic cell is Escherichia coli BL21 cell or Escherichia coli DH5α competent cell.

In a fifth exemplary embodiment of the present application, a method for generating chiral alcohol is provided. The method includes a step of using any one of the above ketoreductase mutants to perform a reduction reaction on the chiral ketone compound represented by the previous structural formula (I) to generate the chiral alcohol. Since the above ketoreductase mutant of the present invention has the good catalytic activity and stereoselectivity for the chiral ketone compound represented by the structural formula (I), the chiral alcohol prepared by using the ketoreductase mutants of the present invention may increase the reaction rate, improve the substrate concentration, reduce the amount of the enzyme, and reduce the difficulty of post-treatment.

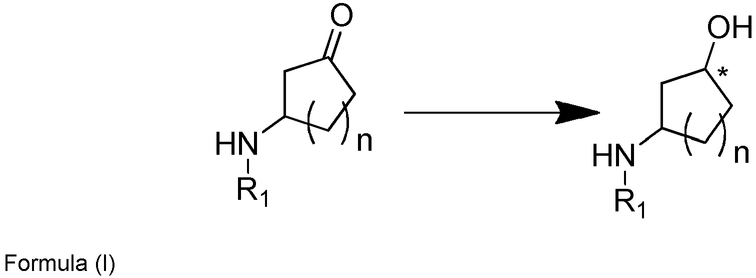

Herein, n is 0, 1, 2 or 3; R1 is selected from: H, OH, a substituted or unsubstituted C1-10 alkyl, a substituted or unsubstituted C3-6 cycloalkyl, a substituted or unsubstituted C6-12 aryl, a substituted or unsubstituted C3-6 heterocyclyl; or, R1 is a benzene ring together with a linked heterocyclic ring to form a fused ring system.

In some preferred embodiments, the chiral ketone compound shown in Formula (I) is

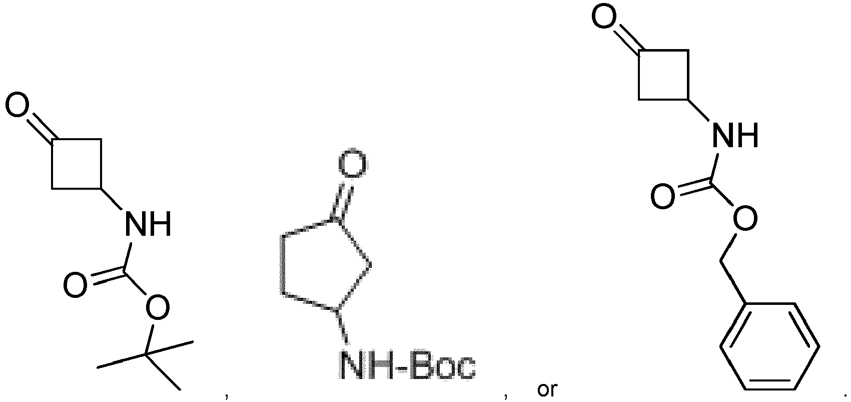

The above ketoreductase mutant of the present application has the higher catalytic activity and stereoselectivity for the above chiral ketone compounds.

In some preferred embodiments, the mass ratio of the ketoreductase mutant to the chiral ketone compound is 0.1-10:1. This mass ratio is used to generate the chiral alcohol compound, due to the high catalytic activity and stereoselectivity of the enzyme, the enzyme amount is small, the substrate conversion rate is high, and the subsequent product separation is easy, so that the production efficiency is high.

In some preferred embodiments, the concentration of the ketone compound in the reduction reaction system is 1 g/L~100 g/L. This substrate concentration is used to generate the chiral alcohol compound, due to the high catalytic activity and stereoselectivity of the enzyme, the enzyme per unit mass may catalyze the conversion of a relatively high concentration of the substrate into the chiral alcohol product, so that the production efficiency is high.

In some preferred embodiments, the reduction reaction is performed at 20~45°C. The reductase mutant of the present application may perform the catalytic reduction reaction of the substrate under relatively room temperature conditions, the production conditions are reduced, and it is more helpful to improve the production efficiency.

The reduction reaction described above generally requires a cofactor, typically a nicotinamide adenine dinucleotide (NADH) or a nicotinamide adenine dinucleotide phosphate (NADPH), and the reduction reaction may include a system for regenerating the cofactor, such as a D-glucose, a coenzyme NAD+, and a glucose dehydrogenase (GDH); a formate compound, the coenzyme NAD+ and a formate dehydrogenase (FDH); or isopropanol, the coenzyme NAD+ and an alcohol dehydrogenase (ADH). In some implementation modes using the purified ketoreductase, such cofactors, and optionally such cofactor regeneration systems, may typically be added to a reaction medium along with the substrate and the ketoreductase. Similar to the ketoreductase, any enzymes containing the cofactor regeneration system may be in the form of an extract or a lysate of such cells, or added to a reaction mixture as a purified enzyme. In an implementation scheme using the cell extract or the cell lysate, the cell used to generate the extract or the lysate may be those expressing the enzyme containing only a cofactor regeneration system or containing the cofactor regeneration system and the ketoreductase. In an implementation scheme using a whole cell, the cell may be those expressing the enzyme containing the cofactor regeneration system and the ketoreductase.

Whether the whole cell, the cell extract, or the purified ketoreductase is used, a single ketoreductase may be used, or, optionally, a mixture of two or more ketoreductases may be used.

The reaction system for generating the chiral alcohol by using the ketoreductase to perform the reduction reaction of the chiral ketone compound further includes a coenzyme, a coenzyme regeneration system and a buffer. Herein, the buffer is usually a phosphate buffer, and may also be a Tris-hydrochloric acid buffer, a sodium barbital-hydrochloric acid buffer or a citric acid-sodium citrate buffer according to the actual situation.

The beneficial effects of the present application are further described below with reference to the specific embodiments.

It should be noted that PB in the following embodiments represents the phosphate buffer.

### Embodiment 1:

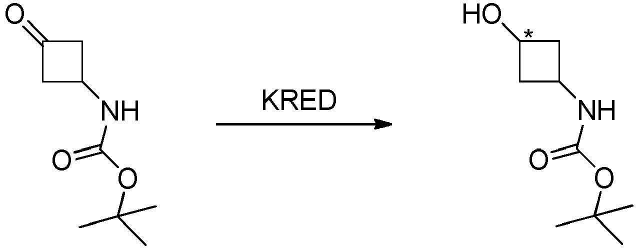

In a 250 mL reaction flask, 500 mg of a raw material is weighed, 16.75 mL of isopropanol is added, 12.5 mL of crude enzyme solution of an AcCR starting enzyme (2.5 g of mutant wet cells are resuspended in 0.1 M PB 7.0, the total volume is 12.5 mL, the crude enzyme solution of which the mass volume content is 20% is prepared by ultrasonication, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to a High Performance Liquid Chromatography (HPLC) to detect a chirality value of a product.

As a result, 40% of the substrate is converted into the product, but the product is lower in chiral purity, and the selectivity is only 39%. It may be seen that the AcCR starting enzyme has a certain catalytic ability, but the selectivity is not good, and the content of the chiral target product is low. Since an isomer may not be separated by a conventional method, the AcCR starting enzyme may not be used in production of the target chiral alcohol.

### Embodiment 2:

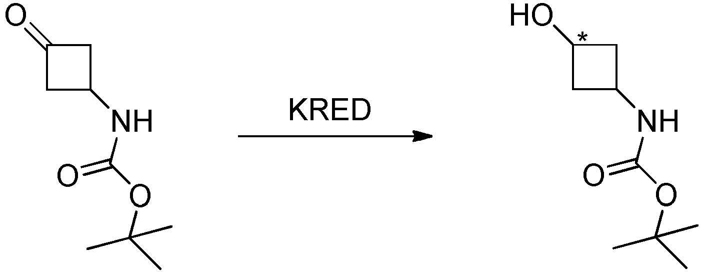

In a 100 mL reaction flask, 500 mg of a raw material is added, 6.7 mL of isopropanol is added, 0.5 mL of crude enzyme solution of an AcCR starting enzyme (0.1 g of wet cells is sonicated to obtain 20% crude enzyme solution, pH=7.0) is added, 100 mM PB 7.0 is added so that the final volume of the system is 10 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product conversion rate and enantiomeric excess (ee) value.

In a 100 mL reaction flask, 500 mg of a raw material is added, 6.7 mL of isopropanol is added, 0.5 mL of crude enzyme solution of an AcCR starting enzyme (0.1 g of mutant wet cells is sonicated to obtain 20% crude enzyme solution, pH=7.0) is added, 100 mM PB 7.0 is added so that the final volume of the system is 10 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product conversion rate and ee value.

Results are shown in Table 5, the enzyme amount (namely, 0.1 g of bacterial wet cells) is further reduced to one-fifth of the mass of the substrate (500 mg), and the volume of the reaction is further reduced to 10 mL. The AcCR starting enzyme and some mutants (due to a large number of the mutants, only some of which are selected) are selected for verification, and the results show that the AcCR starting enzyme is basically inactive, and the selectivity is not high, the target chiral alcohol accounts for only 37%, and the modified mutant not only shows a very high advantage on the selectivity, but also the optimal bacterial selectivity reaches 99%, and the activity is greatly improved, and the conversion is basically completed. Therefore, the transformed mutant shows a great change in catalytic properties, and obtains the extremely high activity and selectivity.

**Table 5:**

| Mutant strain | Mutation site | Activity | Selectiv ity |
|---|---|---|---|
| Starting enzyme | Null | * | * |
| M4 | S96W+L198P | **** | *** |
| M15 | T94S+S96W+I143V+I190T+M194L+L198P+E201D+R206Q | ****** | ***** |
| M19 | T94S+Y95F+S96W+I143V+I190T+M194L+L198P+E201G+R2 06Q | ****** | ***** |
| M34 | T94S+Y95F+S96W+I143V+I190T+M194L+V195I+L198P+E20 1G+R206Q | ****** | ***** |
| M35 | T94S+Y95F+S96W+I143V+I190T+M194L+V195I+L198P+E20 1D+R206Q | ****** | ***** |
| M36 | I35V+T94S+Y95F+S96W+I143V+I190T+M194L+L198P+E201 G+R206Q | **** | ***** |
| M37 | T94S+Y95F+S96W+I143V+V185I+I190T+M194L+L198P+E20 1G+R206Q | ***** | ***** |
| M38 | T94S+Y95F+S96W+I143V+I190T+M194L+L198P+E201G+R2 06Q+T240I | ***** | ***** |
| M39 | T94S+Y95F+S96W+I143V+Y178C+I190T+M194L+V195I+L19 8P+E201D+R206Q | ****** | ***** |
| M40 | T94S+Y95F+S96W+I143V+I190T+M194L+V195I+L198P+E20 1D+A203G+R206Q | ****** | ***** |
| M41 | L198P+S96W+M194L+I143V+R206Q+I190T+E201D+T94S+Y 95F+V195I+A203S | ****** | ***** |
| M42 | T94S+Y95F+S96W+I143V+Y189W+I190T+M194L+L198P+E2 01G+R206Q | ***** | ***** |
| M43 | T94S+Y95F+S96W+M194L+L198P+I143V+R206Q+I190T+E2 01D+D201G+V195I+Y189W | ***** | ***** |
| M44 | T94S+Y95F+S96W+I143V+YI89W+I190T+M194L+V195I+L19 8P+E201D+R206Q | ***** | ***** |
| M45 | T94S+Y95F+S96W+I143V+Y178C+Y189W+I190T+M194L+V1 95I+L198P+E201D+R206Q | ***** | ***** |
| M46 | T94S+Y95F+S96W+I143V+Y189W+I190T+M194L+V195I+L19 8P+E201D+A203G+R206Q | ***** | ***** |
| M47 | T94S+Y95F+S96W+I143V+R206Q+Y189W+I190T+M194L+V 195I+L198P+E201D+A203S | ***** | ***** |
| M48 | T94S+Y95F+S96W+I143V+Y178C+I190T+M194L+V195I+L19 8P+E201D+D202A+A203G+R206Q | ***** | ***** |
| M49 | L198P+T94S+Y95F+S96W+I143V+Y178C+Y189W+I190T+M1 94L+V195I+E201D+D202A+A203G+R206Q | ***** | ***** |

| | | | |
|---|---|---|---|
| In the above table, selectivity: * means that the selectivity is 5%~40%; *** means that the selectivity is 40%~90%; **** means that the selectivity is 90%~95%; and ***** means that the selectivity is 95%~100%. Activity: * means that the conversion rate is less than 10%; **** means that the conversion rate is 10%-70%; ***** means that the conversion rate is 80%-90%; and ****** means that the conversion rate is 90%~100%. | | | |

### Embodiment 3:

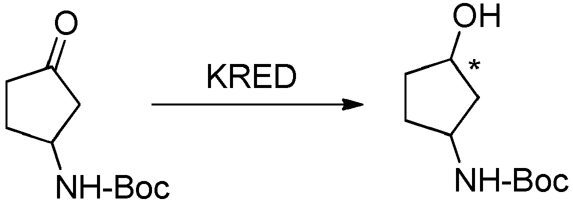

In a 250 mL reaction flask, 500 mg of a raw material is added, 5 mL of isopropanol is added, 12.5 mL of crude enzyme solution of an AcCR starting enzyme (2.5 g of mutant wet cells is sonicated to obtain 20% crude enzyme solution, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product chirality value.

In a 250 mL reaction flask, 500 mg of a raw material is added, 5 mL of isopropanol is added, 12.5 mL of crude enzyme solution of an AcCR mutant (2.5 g of mutant wet cells is sonicated to obtain 20% crude enzyme solution, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product chirality value.

Results are shown in Table 6. It may be seen that compared with the AcCR starting enzyme, the selectivity of the transformed mutant is greatly improved, and an unexpected effect is obtained.

**Table 6:**

| Mutant strain | Mutation site | Selectivity |
|---|---|---|
| Starting enzyme | Null | * |
| M19 | T94S+Y95F+S96W+I143V+I190T+M194L+L198P+E201G+R206 Q | ** |
| M34 | T94S+Y95F+S96W+I143V+I190T+M194L+V195I+L198P+E201G +R206Q | *** |
| M35 | T94S+Y95F+S96W+I143V+I190T+M194L+V195I+L198P+E201D+ R206Q | *** |
| M36 | I35V+T94S+Y95F+S96W+I143V+I190T+M194L+L198P+E201G+ R206Q | *** |
| M39 | T94S+Y95F+S96W+I143V+Y178C+I190T+M194L+V195I+L198P+ E201D+R206Q | *** |
| M40 | T94S+Y95F+S96W+I143V+I190T+M194L+V195I+L198P+E201D+ A203G+R206Q | *** |
| M41 | L198P+S96W+M194L+I143V+R206Q+I190T+E201D+T94S+Y95F +V195I+A203S | *** |
| M42 | T94S+Y95F+S96W+I143V+Y189W+I190T+M194L+L198P+E201 G+R206Q | *** |
| M43 | T94S+Y95F+S96W+M194L+L198P+I143V+R206Q+I190T+E201D +D201G+V195I+Y189W | *** |
| M44 | T94S+Y95F+S96W+I143V+YI89W+I190T+M194L+V195I+L198P+ E201D+R206Q | *** |
| M45 | T94S+Y95F+S96W+I143V+Y178C+Y189W+I190T+M194L+V195I +L198P+E201D+R206Q | *** |
| M46 | T94S+Y95F+S96W+I143V+Y189W+I190T+M194L+V195I+L198P +E201D+A203G+R206Q | *** |
| M47 | T94S+Y95F+S96W+I143V+R206Q+Y189W+I190T+M194L+V195I +L198P+E201D+A203S | *** |
| M48 | T94S+Y95F+S96W+I143V+Y178C+I190T+M194L+V195I+L198P+ E201D+D202A+A203G+R206Q | *** |
| M49 | L198P+T94S+Y95F+S96W+I143V+Y178C+Y189W+I190T+M194 L+V195I+E201D+D202A+A203G+R206Q | *** |

| | | |
|---|---|---|
| In the above table, * means that the selectivity is 50-80%; ** means that the selectivity is 80%~90%; and *** means that the selectivity is 90%~100%. | | |

### Embodiment 4

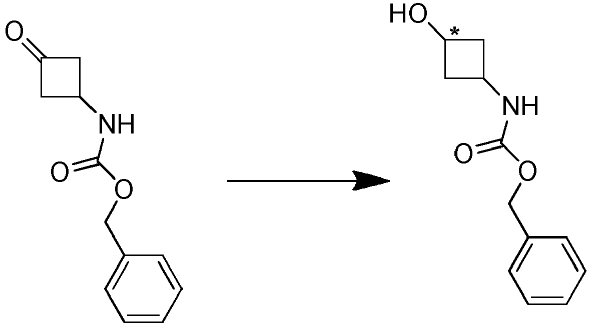

In a 250 mL reaction flask, 500 mg of a raw material is added, 5 mL of isopropanol is added, 12.5 mL of crude enzyme solution of an AcCR starting enzyme (2.5 g of mutant wet cells is sonicated to obtain 20% crude enzyme solution, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product chirality value.

In a 250 mL reaction flask, 500 mg of a raw material is added, 5 mL of isopropanol is added, 12.5 mL of crude enzyme solution of an AcCR mutant (2.5 g of mutant wet cells is sonicated to obtain 20% crude enzyme solution, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product chirality value.

Results are shown in Table 7. It may be seen that compared with the AcCR starting enzyme, the selectivity of the transformed mutant is greatly improved, and an unexpected effect is obtained.

**Table 7:**

| Mutant strain | Mutation site | Selectivity |
|---|---|---|
| Starting enzyme | Null | * |
| M19 | T94S+Y95F+S96W+I143V+I190T+M194L+L198P+E201G+R206 | ** |
| | Q | |
| M34 | T94S+Y95F+S96W+I143V+I190T+M194L+V195I+L198P+E201 G+R206Q | *** |
| M35 | T94S+Y95F+S96W+I143V+I190T+M194L+V195I+L198P+E201 D+R206Q | *** |
| M36 | I35V+T94S+Y95F+S96W+I143V+I190T+M194L+L198P+E201G +R206Q | *** |
| M39 | T94S+Y95F+S96W+I143V+Y178C+I190T+M194L+V195I+L198 P+E201D+R206Q | *** |
| M40 | T94S+Y95F+S96W+I143V+I190T+M194L+V195I+L198P+E201 D+A203G+R206Q | *** |
| M41 | L198P+S96W+M194L+I143V+R206Q+I190T+E201D+T94S+Y95 F+V195I+A203S | *** |
| M42 | T94S+Y95F+S96W+I143V+Y189W+I190T+M194L+L198P+E201 G+R206Q | *** |
| M43 | T94S+Y95F+S96W+M194L+L198P+I143V+R206Q+I190T+E201 D+D201G+V195I+Y189W | *** |
| M44 | T94S+Y95F+S96W+I143V+YI89W+I190T+M194L+V195I+L198P +E201D+R206Q | *** |
| M45 | T94S+Y95F+S96W+I143V+Y178C+Y189W+I190T+M194L+V19 5I+L198P+E201D+R206Q | *** |
| M46 | T94S+Y95F+S96W+I143V+Y189W+I190T+M194L+V195I+L198 P+E201D+A203G+R206Q | *** |
| M47 | T94S+Y95F+S96W+I143V+R206Q+Y189W+I190T+M194L+V19 5I+L198P+E201D+A203S | *** |
| M48 | T94S+Y95F+S96W+I143V+Y178C+I190T+M194L+V195I+L198 P+E201D+D202A+A203G+R206Q | *** |
| M49 | L198P+T94S+Y95F+S96W+I143V+Y178C+Y189W+I190T+M19 | *** |
| | 4L+V195I+E201D+D202A+A203G+R206Q | |

| | | |
|---|---|---|
| In the above table, * means that the selectivity is 40-60%; ** means that the selectivity is 60%~90%; and *** means that the selectivity is 90%~100%. | | |

### Embodiment 5

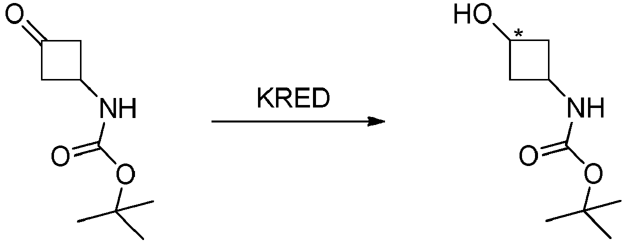

In a 250 mL reaction flask, 500 mg of a raw material is added, 16.75 mL of isopropanol is added, 12.5 mL of crude enzyme solution of an AcCR starting enzyme (2.5 g of mutant wet cells are resuspended in 0.1 M PB 7.0, the total volume is 12.5 mL, and 20% crude enzyme solution is prepared by ultrasonication, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product chirality value.

In a 250 mL reaction flask, 500 mg of a raw material is added, 16.75 mL of isopropanol is added, 12.5 mL of crude enzyme solution of an mutant enzyme (2.5 g of mutant wet cells are resuspended in 0.1 M PB 7.0, the total volume is 12.5 mL, and 20% crude enzyme solution is prepared by ultrasonication, pH=7.0) is added, 100 mM PB 7.0 is added so that the final volume of the system is 25 mL, it is stirred at a constant temperature of 30°C for 16 h, and after the system is centrifuged at 12000 rpm for 5 min, 200 uL of a sample is taken and added to 2 mL of acetonitrile for dissolving, and after being centrifuged at 12000 rpm for 5 min, it is sent to HPLC to detect the product chirality value.

Results show that while it is mutated at the same site and the different types of the amino acids are used, the mutants show greatly different selectivity and activity.

**Table 8:**

| Mutant strain | Selectivity | Activity |
|---|---|---|
| Starting enzyme | * | +++ |
| L198P | *** | ++++ |
| L198T | ** | +++ |
| L198V | * | ++++ |
| L198R | * | ++++ |
| L198D | * | ++ |
| L198Q | ND | ND |
| L198H | ND | ND |
| L198Y | * | + |
| S96W | *** | ++ |
| S96L | *** | +++ |
| S96V | ** | +++ |
| S96F | * | ++ |
| S96I | ** | +++ |
| S96G | * | +++ |
| S96Q | * | ++ |
| S96Y | * | +++ |
| S96M | * | +++ |
| S96H | * | +++ |
| S96P | * | +++ |
| S96N | * | ++ |

| | | |
|---|---|---|
| In the above table, * means that the selectivity is 30-50%; ** means that the selectivity is 50-60%; and *** means that the selectivity is 60-80%. + represents 0-30% of the conversion rate; ++ represents 30-60% of the conversion rate; +++ represents 60-80% of the conversion rate; ++++ represents 80-100% of the conversion rate; ND represents that the activity is not detected. | | |

From the above descriptions, it may be seen that the above embodiments of the present invention achieve the following technical effects: the present application rationally transforms the proteins of the existing reductase mutants by the method combined with rational design and random mutation, and performs the catalytic activity and stereoselectivity screening on the obtained mutants by using the substrate ketones of the present application, finally the mutant strains with high selectivity and high activity are obtained. The strains containing these mutants are applied to the catalytic reduction reaction of the ketone substrates of the present application, so the production efficiency of its corresponding chiral alcohol compounds may be improved.

The above descriptions are only preferred embodiments of the present invention, and are not intended to limit the present invention. For those skilled in the art, the present invention may have various modifications and changes. Any modifications, equivalent replacements, improvements and the like made within the spirit and principle of the present invention shall be included within the scope of protection of the present invention.

## Claims

1. A ketoreductase mutant, having an amino acid sequence of SEQ ID NO:1 with a mutation of one or more amino acids and having a ketoreductase activity: L198P, S96W, L152G, S96W+L198P, S96L+L198P, S96W+M194L+L198P, S96W+Y189F+L198P, S96W+L198P+E201D, S96W+I143V+M194L+L198P+R206Q, S96W+I143V+M194L+L198P+E201D+R206Q, S96W+I143V+I190T+M194L+L198P+E201D+R206Q, T94S+S96W+M194L+L198P, S96W+M194L+L198P+E201G, T94S+S96I+M194L+L198P, T94S+S96W+I143V+I190T+M194L+L198P+E201D+R206Q, S96W+I143V+I190T+M194L+L198P+E201G+R206Q, T94S+S96W+I143V+I190T+M194L+L198P+E201G+R206Q, T94S+Y95F+S96W+I143V+I190T+M194L+L198P+E201D+R206Q, T94S+Y95F+S96W+I143V+I190T+M194L+L198P+E201G+R206Q, S96W+I143V+I190T+M194L+V195I+L198P+E201D+R206Q, S96W+I147V+M194L+L198P, S96W+A153T+M194L+L198P, Q24L+A72V+S101N+S96W+M194L+L198P, S96W+I190T+M194L+L198P+D234V, S96W+M194L+L198P+K237E, S96W+P193H+M194L+L198P, V34M+S96W+M194L+L 198P, S96W+M194L+L198P+E201H, L80R+S96W+M194L+L198P, S96W+M194L+L198P+D234V, S96W+I143V+I190T+M194L+L198P+T199I+E201D+R206Q, S96W+V99T+I143V+I190T+M194L+L198P+E201D+R206Q, S96W+L104S+I143V+I190T+M194L+L198P+E201D+R206Q, T94S+Y95F+S96W+I143V+I190T+M194L+V195I+L198P+E201G+R206Q, T94S+Y95F+S96W+I143V+I190T+M194L+V195I+L198P+E201D+R206Q, I35V+T94S+Y95F+S96W+I143V+I190T+M194L+L198P+E201G+R206Q, T94S+Y95F+S96W+I143V+V185I+1190T+M194L+L198P+E201G+R206Q, T94S+Y95F+S96W+I143V+I190T+M194L+L198P+E201G+R206Q+T24I, T94S+Y95F+S96W+I143V+Y178C+I190T+M194L+V195I+L198P+E201D+R206Q, T94S+Y95F+S96W+I143V+I190T+M194L+V195I+L198P+E201D+A203G+R206Q, S96W+M194L+I143V+R206Q+I190T+L198P+E201D+T94S+Y95F+V195I+A203S, T94S+Y95F+S96W+I143V+Y189W+I190T+M194L+L198P+E201G+R206Q, T94S+Y95F+S96W+M194L+L198P+I143V+R206Q+I190T+E201D+D201G+V195I+Y189W, T94S+Y95F+S96W+I143V+Y189W+I190T+M194L+V195I+L198P+E201D+R206Q, T94S+Y95F+S96W+I143V+Y178C+Y189W+I190T+M194L+V195I+L198P+E201D+R206Q, T94S+Y95F+S96W+I143V+Y189W+I190T+M194L+V195I+L198P+E201D+A203G+R206Q, T94S+Y95F+S96W+I143V+R206Q+Y189W+I190T+M194L+V195I+L198P+E201D+A203S, T94S+Y95F+S96W+I143V+Y178C+I190T+M194L+V195I+L198P+E201D+D202A+A203G +R206Q, or T94S+Y95F+S96W+I143V+Y178C+Y189W+I190T+M194L+V195I+L198P+E201D+D202A +A203G+R206Q.

2. A DNA molecule, wherein the DNA molecule encodes the ketoreductase mutant of claims 1.

3. A recombinant vector, wherein the recombinant vector is linked with the DNA molecule of claim 2.

4. A host cell, wherein the host cell comprises the recombinant vector of claim 3.

5. The host cell according to claim 4, wherein the host cell comprises a prokaryotic cell or an eukaryotic cell.

6. The host cell according to claim 5, wherein the prokaryotic cell is a cell of E. coli.

7. A method for producing a chiral alcohol, wherein the method comprises a step of applying the ketoreductase mutant of claims 1 to catalyze a reduction reaction of a chiral ketone compound to produce a chiral alcohol, the chiral ketone compound is

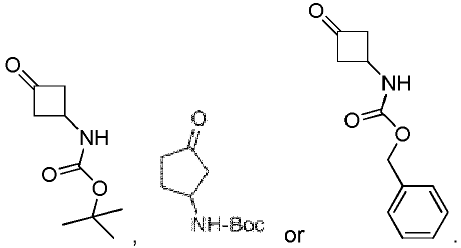

8. The method according to claim 7, wherein a mass ratio of the ketoreductase mutant to the chiral ketone compound is 0.1 to 10: 1.

9. The method according to claim 7, wherein a concentration of the ketone compound in the reduction reaction system is 1 g/L to 100 g/L.

10. The method according to claim 7, wherein the reduction reaction is performed at 20°C to 45 °C.
